# EUROPÄISCHE PATENTSCHRIFT

(11) **EP 1 086 198 B1**
(45) Veröffentlichungstag und Bekanntmachung des Hinweises auf die Patenterteilung: **04.05.2005**
(21) Anmeldenummer: 99955284.7
(22) Anmeldetag: 21.05.1999
(51) Int. Cl.: C11D 1/83, C11D 17/00, A61K 7/50

(54) **DUSCHGEL**
SHOWER GEL
GEL DOUCHE

(30) Priorität: 30.05.1998 DE 19824359
(43) Veröffentlichungstag der Anmeldung: 28.03.2001
(73) Patentinhaber: Henkel Kommanditgesellschaft auf Aktien, 40589 Düsseldorf (DE)
(72) Erfinder: BITTER, Ingrid, D-47800 Krefeld (DE); SCHOLZ, Wolfhard, D-47829 Krefeld (DE)
(86) Internationale Anmeldenummer: PCT/EP1999/003494
(87) Internationale Veröffentlichungsnummer: WO 1999/063032

(56) Entgegenhaltungen:
- EP-A- 0 217 250
- EP-A- 0 681 832
- WO-A-94/16680
- DE-A- 3 714 455
- GB-A- 2 143 841
- US-A- 5 393 450
- PATENT ABSTRACTS OF JAPAN vol. 018, no. 400 (C-1230), 26. Juli 1994 (1994-07-26) & JP 06 116118 A (AJINOMOTO CO INC), 26. April 1994 (1994-04-26)

## Beschreibung

Die Erfindung betrifft ein gelförmiges Körperreinigungsmittel, das sich aus einer flexiblen Kunststoffflasche oder aus einem Pumpspender durch leichten Druck entnehmen läßt, aber im Ruhezustand auf der Handfläche nicht fließt und von der Körperoberfläche nicht abläuft.

Flüssige Körperreinigungsmittel auf Basis von stark schäumenden, synthetischen Tensiden und Tensidgemischen haben die stückförmige Toilettenseife nicht nur wegen der höheren Beständigkeit gegenüber den Härtebildnern des Wassers, sondern auch wegen ihrer leichten Verteilbarkeit auf der Haut und wegen ihres neutralen oder schwach sauren pH-Wertes und der damit verbundenen besseren Hautverträglichkeit weitgehend verdrängt. Die Produkte werden in der Regel auf Basis anioscher Tenside oder von Gemischen aus anionischen und zwitterionischen Tensiden formuliert und durch Zusätze von lipophilen nichtionischen Tensiden. z.B. von Fettsäurealkanolamiden und von Elektrolytsalzen in der Viskosität so angehoben, daß sie nicht vorzeitig von der Hand oder von der Haut ablaufen, sondern sich leicht verteilen lassen. Durch Zusatz von Ölkomponenten und Duftstoffölen werden solche Systeme leicht in ihrer Struktur gestört, so daß die Viskosität absinkt und die Ölkomponenten zur Trübung und Separation führen.

Aus DE 37 14 455 A1 ist z.B. ein gelförmiges Schaumbadkonzentrat bekannt, das aber einen sehr hohen Tensidgehalt von mehr als 45 Gew.-% aufweist. Aus US 4,668,422 ist ein flüssiges Körperreinigungsmittel bekannt, das keine Aniontenside aufweist. Aus DE 35 34 733 A1 ist eine stark schäumende Tensidzubereitung bekannt, die einen hohen Gehalt an anionischen Tensiden und solubilisierten Ölkomponenten aufweist, aber bezüglich der rheologischen Eigenschaften noch nicht voll befriedigt.

Es bestand daher die Aufgabe, ein gelförmiges Körperreinigungsmittel vorzuschlagen, das sowohl einen deutlichen Anteil an stabil emulgierten oder solubilisierten Ölkomponenten als auch eine gelförmige Struktur aufweist, dabei sollte der Tensidgehalt und die Art der Tenside so gewählt werden, daß auch bei unverdünnter Anwendung auf der Haut oder Schleimhaut keine Reizung oder übermäßige Entfettung beobachtet wird.

Diese Aufgabe wurde gelöst durch ein gelförmiges, wäßriges Reinigungsmittel, das einen Gehalt von
(A) 5 - 20 Gew.-% ionischer, wasserlöslicher Tenside
(B) 5 - 15 Gew.-% nichtionischer hydrophiler, wasserlöslicher Tenside
(C) 5 - 10 Gew.-% nichtionischer lipophiler, wasserdispergierbarer Tenside
(D) 0,5 - 2 Gew.-% solubilisierter, wasserunlöslicher Ölkomponenten
und einen Gesamtgehalt an Tensiden (A + B + C) von 20 - 30 Gew.-% sowie ein Gewichtsverhältnis von Ölkomponenten zu Tensiden (D) : (A + B + C) von 1 : 10 bis 1 : 50 aufweist.

Als gelförmig werden dabei solche wäßrigen Zubereitungen verstanden, die ein stark strukturviskoses Verhalten, eine Viskosität bei 25° C von wenigstens 5 Pas bei einer Schergeschwingkeit von 10s⁻¹ und eine Fließgrenze von wenigstens 5 Pa aufweisen. Solche Zubereitungen lassen sich zwar aus Pumpspendern, flexiblen Kunststoffbehältern oder Tuben durch leichten Druck auf die Behälterwand entnehmen, sind aber im Ruhezustand, d.h. ohne Einwirkung von Scherkräften, wenig fließfähig und laufen in kleinen Portionen von senkrechten Flächen nicht ab.

Unter dem Einfluß von Scherkräften nimmt die Viskosität jedoch stark ab, so daß sich solche Zubereitungen sehr gut mit der Hand auf der Körperoberfläche oder auf dem Haar verteilen lassen.

Als ionische wasserlösliche Tenside (A) eignen sich bevorzugt anionische, stark schäumende Tenside sowie zwitterionische Tenside (Betaintenside). Geeignete anionische Tenside sind vor allem Alkylsulfate in Form ihrer Alkanolammoniumsalze und Alkylethersulfat-Tenside. Eine weitere Gruppe sehr geeigneter anionischer Tenside ist die der Ethercarboxylate. Ganz allgemein lassen sich die bevorzugt geeigneten anionischen Tenside durch die allgemeine Formel I

R¹-O-(CH₂-CH₂-O)ₓ-A⁽⁻⁾ M⁽⁺⁾ (I)

darstellen, in der R' eine Alkylgruppe mit 12 - 18 C-Atomen oder eine Gruppe R¹COOCH₂CH₂- oder R¹-CONH-CH₂-CH₂-, x = 0 oder 1 - 4, A eine Gruppe - SO₃⁻ oder - CH2-COO⁽⁻⁾, M⁽⁺⁾ ein Alkali-, Magnesium-, Ammonium- oder Alkanolammonium-Ion und R¹ eine Alkylgruppe mit 12 - 18 C-Atomen ist.

Besonders bevorzugt sind solche erfindungsgemäßen Reinigungsmittel, in denen als ionische Tenside Ethersulfat- und Ethercarboxylat-Natriumsalze im Gewichtsverhältnis 1 : 2 bis 2 : 1 enthalten sind.

Geeignete Ethersulfat-Tenside sind im Handel erhältlich, z.B. unter dem Warenzeichen Texapon®N. Geeignete Ethercarboxylat-Tenside sind ebenfalls im Handel, z.B. unter dem Warenzeichen Akypo®.

Nichtionische hydrophile, wasserlösliche Tenside (B) sind solche, die als lipophile Gruppe einen bevorzugt linearen Alkyl- oder Acylrest mit 8 - 22 C-Atomen und als hydrophile Gruppe einen Glycosidrest, einen Polyglycerinrest, einen Sorbitanrest oder einen Polyglycoletherrest oder mehrere dieser Reste enthalten, und deren HLB-Wert wenigstens bei 10 oder darüber liegt. Als HLB-Wert wird dabei ein Wert verstanden, der sich aus der Beziehung

HLB = 0.2 [100 - L]

ergibt, worin L der Gehalt an lipophilen Alkyl- oder Acylgruppen in Gew.-% des Gesamtmolekulargewichts bedeutet.

Geeignete hydrophile wasserlösliche Tenside sind z.B. Fettalkoholpolyglycolether, z.B. auf Basis von C₁₂-C₁₆-Fettalkoholen mit (im Mittel) 10 - 30 Glycolethergruppen, Alkyl-(oligo)-glycoside, z.B. auf Basis von C₈-C₁₄-Alkylgruppen mit (im Mittel) 1 - 4 Glycosidresten, Ethylenoxidanlagerungsprodukte an Sorbitanfettsäuremonoester oder Fettsäuremonoester von Glycerinoxethylaten.

Als nichtionische lipophile, wasserdispergierbare Tenside (C) werden erfindungsgemäß solche Tenside verstanden, die als lipophile Gruppe eine Alkyl- oder Acylgruppe mit 8 - 22 C-Atomen und als hydrophile Gruppe einen Glycerylrest, einen Sorbitanrest oder einen Rest der Formel - O(CₙH₂ₙO)ₓ - H oder - NH(C₂H₂ₙO)ₓ - H oder - N(C₂H₂ₙOH)₂, worin n = 2 oder 3 ist und x einen Mittelwert von 1 - 3 hat.

Bevorzugte lipophile Tenside sind Anlagerungsprodukte von 1 - 3 Mol Ethylenoxid an C₁₀-C₁₈-Fettalkohole, bevorzugt an C₁₂-C₁₆-Fettalkohole, deren Anteil an Homologen mit 1 bis 3 angelagerten Oxyethylgruppen wenigstens 70 Gew.-% des Homologengemisches ist. Solche Produkte sind im Handel erhältlich, z.B. Arlypon®F (Henkel KGaA). Weitere bevorzugt geeignete lipophile Tenside sind Fettsäurealkanolamide. Kokosfettsäuremonoethanolamid ist z.B. im Handel als Comperlan® LS (Henkel KGaA) erhältlich.

Die erfindungsgemäß geeigneten lipophilen nichtionischen Tenside sind in Wasser zu weniger als 1 Gew.-% löslich, d.h. bei 20° C stellen die 1 %igen wäßrigen Zubereitungen trübe Dispersionen dar. Ihre Löslichkeit steigt aber in Gegenwart wasserlöslicher Tenside stark an. Ihr HLB-Wert liegt - nach der oben angegebenen Beziehung - unterhalb von 10, bevorzugt unterhalb von 5.

Als wasserunlösliche Ölkomponenten (D), die in den erfindungsgemäßen, gelförmigen Reinigungsmitteln bevorzugt klar solubilisiert vorliegen, können sowohl Duftstoffkomponenten bzw. ätherische Öle als auch schwerflüchtige, hautweichmachende Ölkomponenten. Fette oder Wachse oder Gemische solcher Komponenten enthalten sein.

Bevorzugt sind kosmetische Öle, die bei + 20° C noch flüssig sind, besonders gut für die erfindungsgemäßen gelförmigen Reinigungsmittel geeignet.

Besonders bevorzugte Ölkomponenten sind z.B. Isopropylmyristat, Isopropylpalmitat, Isopropylstearat, 2-Hexyldecanol, 2-Octyl-dodecanol, Oleylalkohol, Kohlenwasserstoffe wie z.B. 1,3-Diisooctylcyclohexan oder Paraffinöle und deren Gemische mit Duftstoffölen bzw. ätherischen Ölen.

Bevorzugt wird der Gehalt an Tensiden möglichst niedrig gehalten, so daß in den erfindungsgemäßen Reinigungsmitteln bevorzugt wenigstens 60 Gew.-% Wasser enthalten ist.

Bevorzugt sind als nichtionische hydrophile, wasserlösliche Tenside (B) Alkyl-(oligo)-glucoside (B1) und Monofettsäureester von Glycerinoxethylaten oder Oxethylate von Glycerinmonofettsäureestern (B2) im Gewichtsverhältnis B1 : B2 = 1 : 2 bis 2 : 1 enthalten.

Schließlich können die erfindungsgemäßen gelförmigen Reinigungsmittel die für solche Zubereitungen üblichen Hilfs- und Zusatzmittel in den üblichen Mengen, bevorzugt in einer Gesamtmenge bis höchstens 5 Gew.-%, enthalten. Solche üblichen Hilfsmittel sind z.B. Konservierungsstoffe wie z.B. Natriumbenzoat. Salicylsäure, pH-Wert-Stellmittel wie z.B. Citronensäure oder Natriumcitrat, konditionierende Zusätze für Haarwaschmittel, z.B. kationische, wasserlösliche Polymere, Proteinderivate, Panthenol, Komplexbildner, Farbstoffe, Trübungs- oder Perlglanzmittel. Pflanzenauszüge, Antischuppenwirkstoffe, antimikrobielle oder desinfizierende Zusätze und Elektrolytsalze, z.B. Kochsalz zur Einstellung der Viskosität.

Die erfindungsgemäßen gelförmigen Reinigungsmittel eignen sich insbesondere als Duschgel oder Hautwaschgel zur Verpackung in Tuben und flexiblen Kunststoffbehältern, aus denen sie sich durch Druck auf die Wandung leicht dosieren lassen. Sie zeichnen sich durch eine gute Haut- und Schleimhautverträglichkeit, und ein besonders gutes und feinblasiges Schäumvermögen aus.

Die Herstellung erfolgt bevorzugt, indem man die wasserunlöslichen Ölkomponenten mit dem lipophilen und dem hydrophilen nichtionischen Tensid vermischt und die Mischung gegebenenfalls bis zur Homogenität unter Rühren erwärmt und in die wäßrige Lösung der ionischen Tenside und der anderen wasserlöslichen Komponenten unter Rühren einarbeitet.

Die folgenden Beispiele sollen den Erfindungsgegenstand näher erläutern:

### Beispiele

Es wurden folgende Duschgel-Formulierungen hergestellt:

| | **1** | **2** |
|---|---|---|
| Texapon N70 | 7 (5) | 10 (7) |
| Akyposoft KA 250 BVC | 10 (5) | 10 (5) |
| Plantacare 1200 UP | 8 (4) | 8 (4) |
| Cetiol HE | 6 | 6 |
| Eumulgin HRE 455 | 0,5 | 0,2 |
| Arlypon F | 8 | 8 |
| Isopropylmyristat | 1 | 1 |
| Parfümöl | 1 | 1 |
| Conditioner P7 | 0,66 (0,06) | 0,8 (0,08) |
| Natriumchlorid | 1,0 | 1,0 |
| Citronensäure | 0,25 | 0,25 |
| Na-Benzoat | 0,4 | 0,4 |
| Salicylsäure | 0,2 | 0,2 |
| Wasser | ad 100 | ad 100 |
| Viskosität (25° C) [Pa · s] d = 10 s⁻¹ | 8,8 | 16,7 |
| Fließgrenze (25° C) [Pa] | 6,5 | 6,5 |

Die Angaben beziehen sich auf Gew.-% telquel, bei wasserhaltigen Rohstoffen ist in Klammern der Anteil an Gew.-% Aktivsubstanz angegeben.

Die Viskosität wurde gemessen bei 25°C mit einem Haake-Viskosimeter RS 155 (Kegel-Platte-Meßsystem).

Es wurden die folgenden Rohstoffe (Handelsprodukte) eingesetzt:

| | |
|---|---|
| Texapon®N 70 (Henkel KGaA) | Fettalkohol-(C₁₂/C₁₄)-polyglycolether-(2EO)-sulfat, Na-Salz |
| Akyposoft®KA 250 BVC (Chem Y) | Kokosfettsäureamid-polyglycolether-(6EO)-carboxylat, Na-Salz |
| Plantacare®1200 UP (Henkel KGaA) | Alkyl-(C₁₂/C₁₄)-oligo-(1,4)-glucosid |
| Cetiol®HE (Henkel KGaA) | Glycerinpolyglycolether-(7,4EO)-kokosfettsäure-(C₈-C₁₈)-monoester |
| Eumulgin®HRE 455 (Henkel KGaA) | geh. Ricinusölpolyglycolether-(40EO), Lösung in Propylenglycol / H₂O |
| Arlypon® F (Henkel KGaA) | Fettalkohol-(C₁₂/C₁₄)-polyglycolether (2,5EO, narrow range) |
| Conditioner P7 (Sigma) | Acrylamid-Dimethyldiallylammoniumchlorid-Copolymer |

### Herstellung der Duschgel-Beispiele

Isopropylmyristat, Parfümöl, Eumulgin HRE 455 und Cetiol HE wurden gemischt. Die übrigen Komponenten wurden der Reihe nach in Wasser gelöst. Schließlich wurde die Mischung der Ölkomponenten und die wasserfreien Tenside in die wäßrige Lösung eingearbeitet. Zuletzt wurde das Arlypon F zugesetzt und durch Na-Cl die Viskosität eingestellt.

## Patentansprüche

1. Gelförmiges, wäßriges Reinigungsmittel, enthaltend
(A) 5 - 20 Gew.-% ionische, wasserlösliche Tenside
(B) 5 - 15 Gew.-% nichtionische, hydrophile, wasserlösliche Tenside
(C) 5 - 10 Gew.-% nichtionische, lipophile, wasserdispergierbare Tenside
(D) 0,5 - 2 Gew.-%, wasserunlösliche Ölkomponenten,
**dadurch gekennzeichnet, daß** die Ölkomponenten solubilisiert vorliegen und der Gesamtgehalt an Tensiden (A + B + C) von 20 - 30 Gew.-% und das Gewichtsverhältnis von Ölen zu Tensiden (D) : (A + B + C) = 1 : 10 bis 1 : 50 beträgt, wobei das Mittel bei 25°C eine Viskosität von wenigstens 5 Pa·s bei einer Schergeschwindigkeit von 10 s⁻¹ und eine Fließgrenze von wenigstens 5 Pa aufweist..

2. Gelförmiges, wäßriges Reinigungsmittel nach Anspruch 1, **dadurch gekennzeichnet, daß** wenigstens 60 Gew.-% Wasser darin enthalten sind.

3. Gelförmiges, wäßriges Reinigungsmittel nach einem der Ansprüche 1 oder 2, **dadurch gekennzeichnet, daß** als ionische Tenside Ethersulfat- und Ethercarboxylat-Natriumsalze im Gewichtsverhältnis 1 : 2 bis 2 : 1 enthalten sind.

4. Gelförmiges, wäßriges Reinigungsmittel nach einem der Ansprüche 1 bis 3, **dadurch gekennzeichnet, daß** als hydrophile wasserlösliche Tenside Alkyl-(oligo)-glycoside und Monofettsäureester von Glycerinoxethylaten oder Oxethylaten von Glycerinmonofett-säureestern im Gewichtsverhältnis 1 : 2 bis 2 : 1 enthalten sind.

5. Gelförmiges, wäßriges Reinigungsmittel nach einem der Ansprüche 1 bis 4, **dadurch gekennzeichnet, daß** als weitere Komponenten Elektrolytsalze, kationische, haarkonditionierende Polymere und antimikrobielle, konservierende Stoffe enthalten sind.

## Claims

1. An aqueous cleaning agent in gel form, containing
(A) 5 - 20% by weight of ionic, water-soluble surfactants
(B) 5 - 15% by weight of non-ionic, hydrophilic, water-soluble surfactants
(C) 5 - 10% by weight on non-ionic, lipophilic, water-dispersible surfactants,
(D) 0.5 - 2% by weight of water insoluble oil components,
**characterized in that** the oil components are solubilized and the total surfactant (A + B + C) content is from 20 - 30% by weight and the weight ratio of oils to surfactants is (D):(A + B + C) = 1:10 to 1:50, wherein the agent has a viscosity at 25°C of at least 5 Pa.s at a shear rate of 10 s⁻¹ and a flow limit of at least 5 Pa.

2. The aqueous cleaning agent in gel form, according to claim 1, **characterized in that** at least 60% by weight of water is contained therein.

3. The aqueous cleaning agent in gel form, according to any of claims 1 or 2, **characterized in that** ethersulfate and ethercarboxylate sodium salts are contained as ionic surfactants in the weight ratio from 1:2 to 2:1.

4. The aqueous cleaning agent in gel form, according to any of claims 1 to 3, **characterized in that** alkyl-(oligo)-glycosides and mono-fatty acid esters of glycerine oxethylates or oxethylates of glycerine mono-fatty acid esters are contained as hydrophilic water-soluble surfactants, in the weight ratio from 1:2 to 2:1.

5. The aqueous cleaning agent in gel form, according to any of claims 1 to 4, **characterized in that** electrolyte salts, cationic, hair-conditioning polymers and anti-microbial preservatives are contained as further components.

## Revendications

1. Agent de nettoyage aqueux en forme de gel, contenant
(A) 5 - 20% en poids d'agents tensioactifs ioniques solubles dans l'eau
(B) 5 - 15% en poids d'agents tensioactifs non ioniques, hydrophiles, solubles dans l'eau
(C) 5 - 10% en poids d'agents tensioactifs non ioniques, lipophiles, dispersibles dans l'eau
(D) 0,5 - 2% en poids de composants huileux insolubles dans l'eau,
**caractérisé en ce que** les composants huileux se trouvent sous forme solubilisée et la teneur totale en agents tensioactifs (A + B + C) est de 20 - 30% en poids et le rapport pondéral huiles à agents tensioactifs (D) : (A + B + C) = 1 : 10 à 1 : 50, l'agent présentant, à 25°C, une viscosité d'au moins 5 Pa.s à une vitesse de cisaillement de 10 s⁻¹ et une limite d'écoulement d'au moins 5 Pa.

2. Agent de nettoyage aqueux sous forme de gel selon la revendication 1, **caractérisé en ce qu'**au moins 60% en poids d'eau y sont contenus.

3. Agent de nettoyage aqueux sous forme de gel selon l'une quelconque des revendications 1 ou 2, **caractérisé en ce que** sont contenus, comme agents tensioactifs ioniques, des sels de sodium d'éthersulfate et d'éthercarboxylate dans un rapport pondéral de 1 : 2 à 2 : 1.

4. Agent de nettoyage aqueux sous forme de gel selon l'une quelconque des revendications 1 à 3, **caractérisé en ce que** sont contenus, comme agents tensioactifs hydrophiles solubles dans l'eau des alkyl-(oligo)-glycosides et des monoesters d'acides gras d'oxéthylates de glycérol ou des oxéthylates de monoesters d'acide gras de glycérol dans un rapport pondéral de 1 : 2 à 2 : 1.

5. Agent de nettoyage aqueux sous forme de gel selon l'une quelconque des revendications 1 à 4, **caractérisé en ce que** sont contenus comme autres composants des sels électrolytiques, des polymères cationiques de conditionnement des cheveux et des substances de conservation antimicrobiennes.
